# EUROPEAN PATENT APPLICATION

(11) **EP 2 135 548 A1**
(43) Date of publication of application: **23.12.2009**
(21) Application number: 08075558.0
(22) Date of filing: 18.06.2008
(51) Int. Cl.: A61B 5/04

(54) **Microdrive for use in neurophysiological research of animals**

(71) Applicant: Universiteit van Amsterdam, 1012 WX Amsterdam (NL)
(72) Inventor: Manuputy, Ronald Jozef Domingus, 1121 EL Landsmeer (NL); Pennantz, Cyrille Marie Antoine, 1181 PC Amstelveen (NL)
(74) Representative: van Kooij, Adriaan

(57) **Abstract**

Microdrive for use in neurophysiological research of animals, particulary for use in mice, comprising:
- at least two elongate electrodes for measuring field potentials from nervous tissue;
- electrode adjusting means for adjusting the depth of each of the electrodes in the nervous tissue; and
- a body for holding the electrode adjusting means,

wherein the electrodes comprise upper parts held in the electrode adjusting means and lower parts forming a bundle arranged for insertion in the animal,
wherein the electrodes extend substantially parallel and linear over their entire length.

## Description

The invention relates to a microdrive for use in neurophysiological research of animals, specifically for use in mice, comprising at least two elongate electrodes for measuring field potentials from nervous tissue, electrode adjusting means for adjusting the depth of each of the electrodes in the nervous tissue and a body for holding the electrode adjusting means, wherein the electrodes comprise upper parts held in the electrode adjusting means and lower parts forming a bundle arranged for insertion in the animal.

Such a microdrive is known from international PCT-publication WO 98/41145.

As a major model in molecular and cellular neuroscience, the mouse is becoming increasingly important as an experimental subject for systems neuroscience as well. In the last 15 years, gene targeting techniques have become a major tool for systems neuroscience, particularly in the study of mouse behavior and physiology. Compared to other techniques for manipulating the molecular and neurochemical state of the brain, gene targeting offers greater specificity and uniform distribution of the manipulation across the brain areas of interest. Further technological advances have increased the attractiveness of gene targeting methods, by restricting the manipulation to a targeted brain area, or by temporally controlled, reversible induction, or knockout, of gene expression.

Neural ensemble recording, i.e. the simultaneous recording of the activity of groups of neurons with single-unit isolation, in behaving, freely moving animals, has been a key technique in the study of the neural basis of cognition and behavior. Neural ensemble data have informed our current concepts of some of the most important cognitive domains. A classic example is the spatial cognition and navigation system. Our knowledge of this system is largely based on data about spatial correlates of the activity of cell ensembles in the hippocampal system and related structures of rodents, most frequently rats, and their ensemble dynamics. The results obtained with these techniques have recently been at least partially confirmed by data collected in human patients. Applications of ensemble electrophysiology in rats have been recently spreading to other cognitive domains and other parts of the brain, e.g. the striatum, the medial prefrontal cortex and the orbitofrontal cortex.

So far, neural ensemble recording studies in mice have had some important successes, often in combination with gene targeting, yet the technique has remained significantly more limited in its behavioral applications and cell yields as compared to complementary studies in rats.

One major hurdle in obtaining reliable ensemble recordings, with a sizeable number of monitored units, has been that even the lightest of the currently available recording devices is still heavy and large relative to the tiny size of the mouse. Mouse recording devices in use today are usually scaled-down versions of instruments designed for the rat, an animal about 10 times the size of a mouse and proportionally stronger. With such devices only a few (1-4) independently movable electrodes can be usually implanted, yet they are relatively heavy, often exceeding 10% of the animal's body weight. Even when the implanted mouse is not completely impaired in its free movement throughout an environment, the weight of the electrode drive limits the range of possible behavioral tasks to very basic ones, such as spatial exploration and foraging.

It is an object of the present invention to provide a efficient electrode drive which is light weighted, small in size, which enables precise and independent day-to-day positioning of electrodes, which is compatible with existing, commercially available electrophysiology data acquisition systems, which is simply assembled and/or which is re-usable multiple times.

In order to accomplish that objective, a microdrive of the kind referred to in the introduction is **characterized in that** the electrodes extend substantially parallel and linear over their entire length. Because the path of the electrodes is substantially rectilinear, the friction between adjacent electrodes is minimal when, for instance, adjusting the relative depths of the electrodes. This reduction in friction between the electrodes in the bundle reduces the tendency of the electrode to buckle significantly.

In the known microdrive, targeted for use in rats, the upper parts of the electrodes are inclined outwardly and are bent to form a bundle with the lower parts. Especially in this bending region, the danger of buckling occurs when adjusting the depths of the electrodes. Furthermore, this outwardly inclined configuration of the electrode adjusting means results in an enlarged upper diameter of the microdrive, rendering the microdrive practically useless for mice.

In one preferred embodiment of the microdrive according to the invention, the electrode adjusting means comprise electrode holding pieces for holding the upper part of an electrode and conveying means for conveying the electrode holding pieces over a predetermined height, wherein the electrode holding pieces are provided with at least one outwardly orientated contact surface for abutting another electrode holding piece and/or the body, thereby fixing the orientation of said electrode holding pieces in the plane perpendicular to the axis of the electrodes. In this way, guiding means are provided for the electrode holding pieces without the need for extra parts in the microdrive for aligning the electrode holding pieces, hereby simplifying the design and reducing the weight. The holding pieces themselves and/or the body inhibit substantially any movement other than a movement over the conveying means for adjusting the height or depth of the electrode, in particular movement in the plane perpendicular to the electrodes. In particular, the holding pieces slide along an inner surface of the body when being adjusted.

It should be noted that the term "axial" and "radial" as used herein should be seen with respect to the longitudinal axis of the microdrive, i.e. the axis wherein the electrodes extend substantially rectilinear. Adjusting the height of the electrode holding pieces therefore refers to a movement in the axial direction of the microdrive. To this direction is also referred as a vertical movement, wherein lowering relates to a movement in the direction of the animal in the connected state. The radial plane refers to the plane perpendicular to this axis of the electrodes.

The electrode holding pieces preferably extend radially and adjacent to each other in circle segments around the electrodes. The holding pieces substantially fill the interior of the body around the electrodes which extend through the axis of the body, resulting in a close fit of each of the holding pieces. More preferably, the electrode holding pieces are substantially wedge-shaped, wherein the radially extending side surfaces of the holding pieces are arranged to engage a side surface of the adjacent holding pieces and wherein the circumferential surface which is directed away from the electrodes is arranged to engage the inner surface of the body.

In another preferred embodiment of a microdrive according to the invention, the body substantially surrounds the electrode adjusting means, allowing the electrode holding pieces with their contact surfaces to slide over the inner surface, i.e. the surface directed to the axis of the electrodes, of the body. The body hereby forms an exoskeleton, wherein the electrode adjusting means substantially extend inside the body. Preferably, the body is provided with an open top side for insertion of the electrodes and an aperture on the bottom side to house the electrode bundle. More preferably, the inner surface of the body is arranged to limit the movement of the electrode holding pieces in any direction other than the axial displacement for adjusting the height.

In another preferred embodiment of a microdrive according to the invention, the electrode is held on a radially inward location on the electrode holding piece with respect to and at a distance from the conveying means. This configuration allows the electrode holding pieces to receive the electrodes substantially in the central axis of the microdrive, while at the same time receiving the guiding means, which are typically relatively large compared to the electrode, at a more radial position in the microdrive.

In another preferred embodiment of a microdrive according to the invention, the electrode holding piece is provided with an axially oriented hole for receiving a capillary tubing, wherein the capillary tubing is arranged to receive the electrode. Preferably, the capillary tubing comprises polyimide-coated fused silica capillary tubing. More preferably, the capillary tubing is glued in the hole in the electrode holding piece using for instance cyanoacrylate gel.

In another preferred embodiment of the microdrive according to the invention, the conveying means comprise a screw received by the electrode holding piece, wherein relative rotation of the electrode holding piece and the screw results in the adjustment of the height of the electrode holding piece. The screw functions as guide for the electrode holding piece to travel in axial direction to adjust the height. Preferably, the electrode holding piece is provided with a hole for receiving the screw, wherein the inner surface of the hole is threaded allowing vertical movement upon relative rotation of the holding piece and the received screw.

In one preferred embodiment of the height adjusting means according to the invention, the screw is rotatable accommodated in the body, wherein rotation of the screw around its longitudinal axis results in the adjustment of the height of the electrode holding piece. Preferably the screw is rotatable accommodated in a base of the body extending in a substantially radial plane. More preferably, the base is provided with holes for receiving an unthreaded end of the screw. Using this assembly, height adjusting means are provided using a minimum of moving parts, increasing the reliability of the microdrive. More preferably, the screw is provided with alignment means for substantially limiting movement other than axial rotation. In particular, the alignment means comprise means for limiting the radial movement of the upper side of the screw with respect to the upper part of the body, particularly the core piece as will be explained below. For easy rotation of the screw, the upper part of the screw is provided with a square profile and more preferably is the upper part of the screw provided with a screw piece provided with a square profile.

In another embodiment of the height adjusting means according to the invention, the screw is received by a screw device rotatable accommodated by the electrode holding piece, such that rotation of the screw piece results in the adjustment of the height of the electrode holding piece with respect to the screw. Comparable electrode adjustment means are used in the known microdrive. The screw is preferably anchored to the body, more preferably at a lower portion or base of the body, for inhibiting rotation of the screw around its longitudinal axis. Rotation of the screw devices, which are preferably provided with a threaded hole for engagement on the thread of the screw, hereby results in the movement of the screw device and therewith the electrode holding piece over the screw.

It will be appreciated by the skilled person that both of the embodiments of the height adjusting means render the use of a supporting rod according to prior art adjusting means for preventing rotation of the electrode holding piece when the screw device or screw is rotated redundant, since the contact surfaces on the electrode holding pieces prevent rotation. It will be appreciated that this simplifies the configuration of the microdrive of the current invention. As an example, these electrode adjustment means allow each electrode about 5 mm of useful travel range. Each 360° turn of the screw device or screw piece lowers the electrode by 250 µm.

It should further be noted that the height adjusting means according to the invention can also be used in other microdrives, i.e. microdrives provided with non-linear extending electrodes.

In another preferred embodiment of a microdrive according to the invention, the microdrive comprises a housing, wherein the electrode adjusting means extend outside the housing, allowing adjustment of the depths of the individual electrodes without removing the housing. Preferably, the screw pieces extend outside the housing, such that the for example square profiles can be easily accessed using for instance a rotation tool.

In another preferred embodiment of a microdrive according to the invention, the housing comprises a cap, wherein the cap is arranged to receive the upper ends of the screw devices for easy access and wherein the cap is arranged to be connected to the body, preferably using micro-screws. It is also favorable to provide the microdrive with a core piece, wherein the core piece extends within in the housing and wherein the core piece is arranged to hold the screw devices in place. Preferably, the core piece is connected to the body using micro-screws, more preferably to the upper, open part of the body, wherein the core piece comprises a hole for receiving the upper parts of the electrodes.

In another preferred embodiment of a microdrive according to the invention, the electrodes comprise tetrodes. Preferably, four threads of polymide-insulated, 13 µm diameter nichrome wire (from e.g. Kanthal, PalmCoast, FL) are twisted together and the insulation is melted by heating until the bundle is stably fused together. At one end, the wires are left unbundled so that they can diverge to their electrical contacts, as will be discussed more in detail hereunder.

In another preferred embodiment of a microdrive according to the invention, the microdrive comprises a printed circuit board provided with electrode connectors for connecting with each of the electrodes, wherein the printed circuit board at least partially forms the housing of the microdrive. Using a printed circuit board provided on the outside of the microdrive, whereby the printed circuit board forms at least a part of the housing, saves internal space compared to the internal boards used in known microdrives. Preferably, the exposed surface of the printed circuit board comprises electronic components, such as miniature preamplifiers. It should be noted that this feature of the external printed circuit board is also applicable to other microdrives, for instance without rectilinear extending electrodes. The electrode connectors are preferably provided near the plane perpendicular to the axis of the electrodes wherein the upper ends of the electrodes extend. In this configuration, the electrodes, preferably the tetrodes, which preferably extend through the open top side from the body and the hole provided in the core piece, can be easily connected to the circuit board. Preferably, the connections are established by micro-soldering and covered with conformal coating or nail polish for protection.

In another preferred embodiment of a microdrive according to the invention, the printed circuit board comprises at least one protrusion which protrudes from the microdrive for forming an external connector for the microdrive. Preferably, said connector is compatible to connecting devices known in the art.

In another preferred embodiment of a microdrive according to the invention, a substantially plate shaped, flexible printed circuit board is folded around the body for forming said housing. A flexible printed circuit board or flexprint is thinner and lighter than regular printed circuit boards, resulting in a more weight and size efficient microdrive. Preferably, the connector is formed by at least one protruding flap on the unfolded circuit board, which by folding the flexible printed circuit board around the microdrive forms said connector.

In another preferred embodiment of a microdrive according to the invention, at least the body of the microdrive is manufactured in PEEK, reducing the weight of the microdrive while still achieving a superior strength-to-weight ratio. Preferably, the electrode holding pieces are also manufactured from PEEK.

In another preferred embodiment of a microdrive according to the invention, the microdrive is provided with a base for receiving the bundle of electrodes, wherein the bundle comprises cannulae held in the base for receiving each of the electrodes. The cannula bundle making up the bottom end of the drive is preferably realized by 10 mm pieces of 30-ga hypodermic tubing, and is more preferably held together at the base by a stainless steel ring (for instance 3 mm long, 1.4 mm diameter). It is favorable to place a shorted piece of cannula at the center of the bundle as a spacer.

As an example, the procedure for assembling a microdrive comprising six electrodes according to the invention is described. The electrode holders are inserted on the screws so that they form a hexagon, with the capillaries laying parallel at the center of the hexagon. The cannula bundle is inserted through the base and next into the body; the base is then snapped on the body, so that the outer ring of the bundle protrudes downwards from the bottom of the base and the top ends of the cannulae are exposed inside the body. Next, the silica capillaries are carefully inserted each one in a cannula and the core piece is gently inserted in the body, and secured with 6 micro-screws. The flexible printed circuit board is then wrapped around the drive sides. At this point the top extremity of the capillaries is accessible through the central aperture in the core piece. The insulating layer is removed from the top, unbundled end of the tetrode wire by brief exposure to a flame. Tetrodes can now be threaded through the capillary, and the exposed portion of the wire is inserted in the appropriate connection hole in the printed circuit board. As a last step, the tetrodes are glued to the bottom end of the silica capillaries with cyanoacrylate, and cut to the desired length. The tetrode tips are electrolytically gold-plated in a gold cyanide solution (Select Plating, Meppel, the Netherlands) to an impedance of 0.5-1.0 MΩ and next the tetrodes are retracted so that the tip is flush with the bottom end of the cannulae.

As the electrode holding pieces moves up and down, each of the capillary-tetrode combinations slides inside one of the cannulae. Because the path of the silica capillary is completely rectilinear, and friction is minimal, the need for second hypodermic tubing attached to the electrode holding piece, sliding over another cannula at the bottom end, and preventing the capillary from buckling, is obviated.

The invention will now be explained in more detail with reference to figures illustrated in a drawing, wherein:
- Fig. 1 is a perspective, schematic view of a preferred embodiment of a microdrive according to the invention;
- Fig. 2 is a perspective, schematic cross-sectional view of the microdrive from figure 1;
- Fig. 3 is a schematic view of the body and the electrode holding pieces;
- Fig. 4A is a perspective view of the electrode adjusting means according to the invention;
- Fig. 4B is schematic cross-sectional view of the electrode adjusting means from figure 4A;
- Fig. 5A and 5B are schematic overviews of the configuration of the electrode holding pieces in the body in cross section and perspective respectively;
- Figs. 6A and B are two schematic views of two embodiments of the flexible printed circuit board according to the invention;
- Fig. 7 is a schematic perspective view of a microdrive according to the invention comprising the flexible printed board from figure 6B, and connected to conventional headstages with preamplifiers, and;
- Figs. 8A-C are schematic views of various tools for use with the microdrive according to the invention.

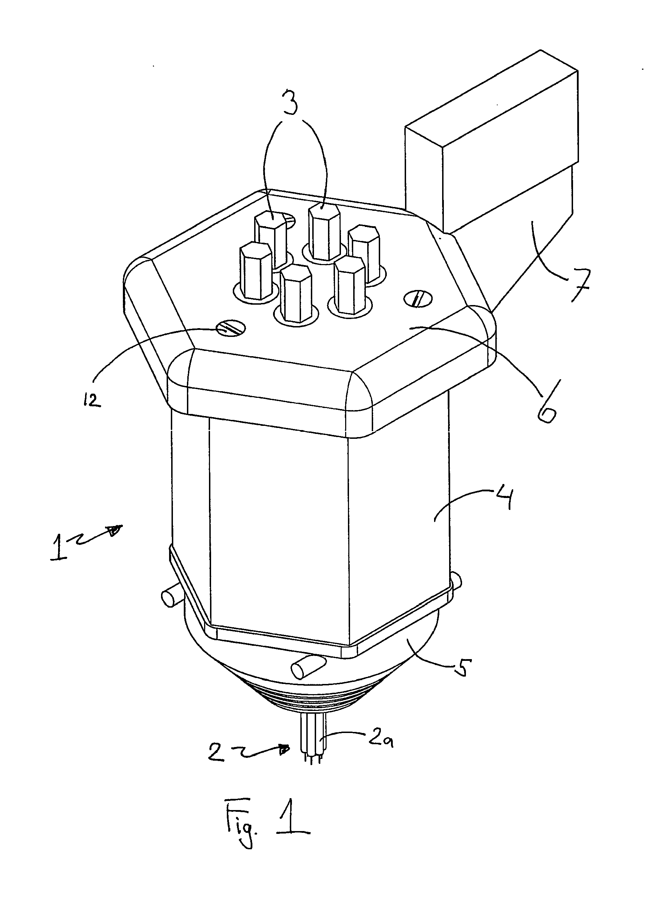

In figure 1, a microdrive 1 according to the invention is shown. The microdrive 1 comprises a housing, which is formed by a cap 6, a flexible printed circuit board (PCB) 4 surrounding an exoskeleton or body 8 (not shown), and base piece 5. The cap 6 is screwed to the microdrive using micro-screws 12 and the base 5 can be snapped on a body piece (not shown) as will be explained more in detail later. The flexible PCB 4 is folded around the body piece to form a hexagonal prism, since the microdrive 1 is arranged to contain six tetrodes 2a. Said tetrodes 2a extend from a hole in the base piece 5 and form a bundle 2 for insertion in the animal. In order to be able to adjust the depths of each of the tetrodes 2a, six screw pieces 3 of the electrode adjusting means for each of the tetrodes 2a extend through the cap 6. The top surfaces of the screw pieces 3 comprise a hexagonal top, facilitating the rotation of said screw pieces 3 for adjusting the depths with a rotation tool. The microdrive 1 furthermore comprises a connector 7 for connecting the microdrive 1 to for instance a conventional data-acquisition system.

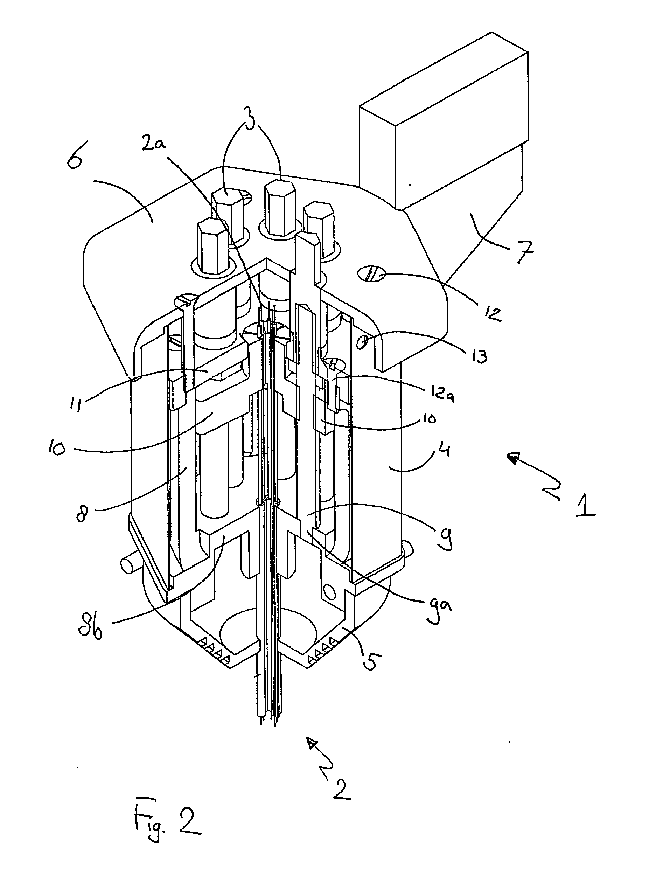

The internal configuration of the microdrive 1 is in more detail shown in figure 2, and is substantially formed by body 8. The body 8 forms an exoskeleton for holding the electrode adjusting means, which comprise screws 9, electrode holding pieces 10 and said screw pieces 3. The unthreaded lower parts 9a of screws 9 are accommodated in holes in bottom part 8b of the body 8 to allow rotation of screws around their longitudinal axis. The body piece 8 has an open top side and an aperture in the bottom part 8b for receiving the bundle 2. On the open top side of the body piece 8 a core piece 11 is provided which is secured on the body piece 11 using micro-screws 12a. The screw pieces 3 are held in place by holes provided in core piece 11. Cap 6 is screwed to core piece 11 using micro-screws 12. The core piece 11 is furthermore arranged to receive the bundle 2 extending from the base piece 5, through the body piece 8 and through the core piece 11 in a rectilinear path. In the region between the core piece 11 and the cap 6, the upper ends of the electrodes forming the tetrodes 2a can be connected (not shown) to connectors 13 on the PCB 4.

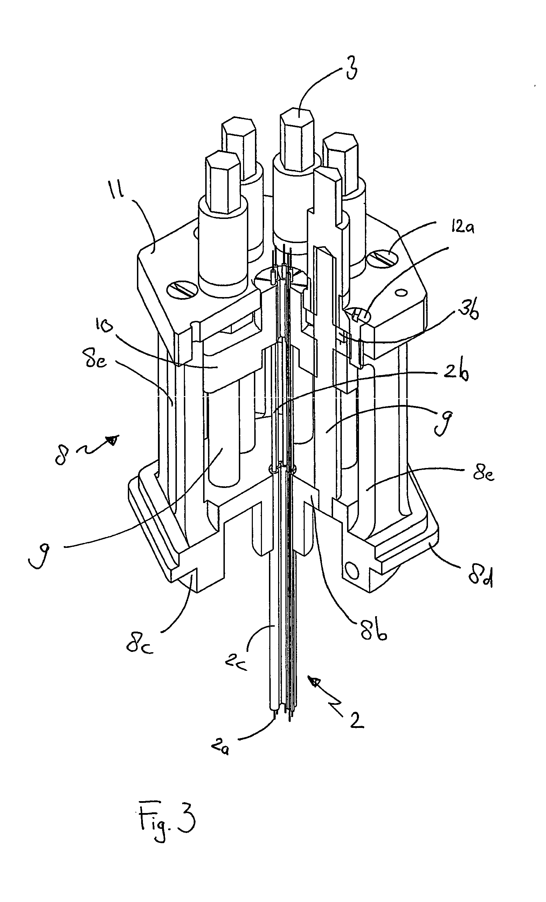

It will be appreciated that the microdrive 1 according to the invention has a simple configuration, mainly consisting of four structural elements, namely the body piece 8, the core piece 11, the cap 6 and the base piece 5. As is more clearly shown in figure 3, the body piece 8 comprises a rim 8c allowing a snap-fit connection of the base piece 5 and rims 8d for receiving the flexible PCB 4, allowing the latter to be easily folded over the body piece 8. In order to save weight, the body piece 8 can be provided with apertures 8e. As is mentioned earlier, the body piece 8 forms an exoskeleton for holding the six electrode adjusting means. The screws 9 of the electrode adjusting means extend in a circular fashion in the bottom part 8b of the body, on a radial position from the bundle 2 extending in the axis of the microdrive 1.

The bundle 2 in this embodiment is formed by six tetrodes 2a provided with capillaries 2b which are glued to the electrode holding pieces 10. Near the aperture in the bottom part 8b of the body 8, the capillaries 2b containing the tetrodes 2a are inserted into cannulae 2c.

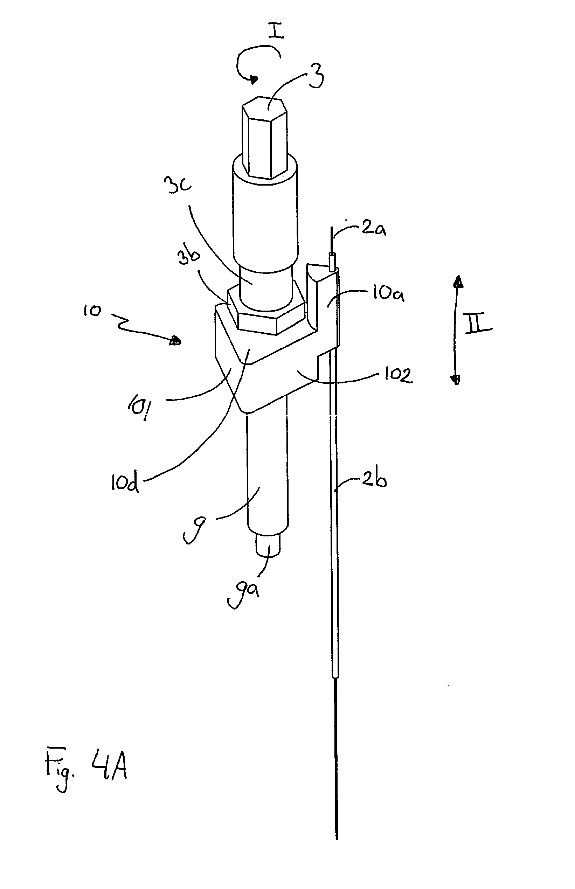

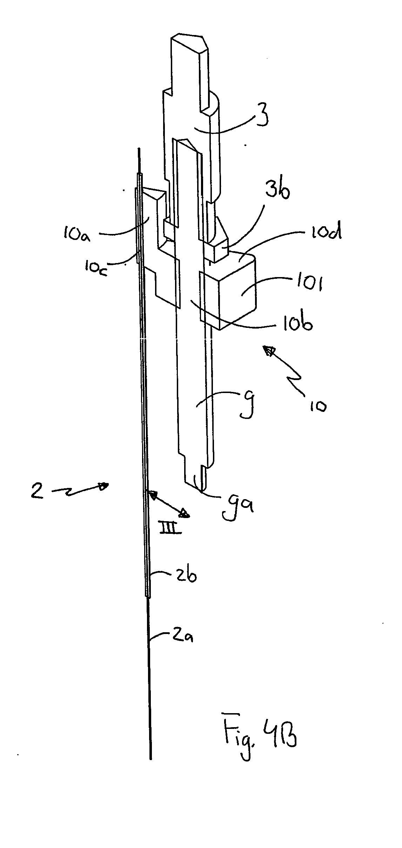

In figure 4A and 4B the height adjusting means according to the invention are shown in more detail. The electrode holding piece 10 is substantially wedge-shaped, comprising circumferential surface 101, with respect to the axis of tetrodes 2a, and radially extending surfaces 102. The part 10a of the holding piece 10 extending towards the axis of the microdrive 1 is provided with a axially orientated hole 10c for receiving the capillary 2b containing the tetrode 2a. The capillary 2b is glued inside hole 10c. For an enhanced connection between the capillary 2b and the holding piece 10, the height of the part 10a if larger than the height of the main body 10d of the holding piece 10.

The main body 10d is provided with a hole 10b for receiving the screw 9. The inner surface of hole 10b is threaded allowing vertical movement (indicated by arrow II) when screw piece 3 is rotated in direction I. As can be seen more clearly in figure 4B, the electrode 2 is held on a radially inward location on a distance III from the conveying means, i.e. the screw 9. This distance III allows the compact assembly of the height adjusting means as will be explained below.

Furthermore, is the upper side of screw 9 provided with said screw piece 3, whereby the screw piece 3 is glued onto screw 9. Also provided are alignment means in the form of bolt 3b and screw piece part 3c for limiting the movement of the screw 9 in a direction other than rotation I. The reduced diameter of screw piece part 3c corresponds to the diameter of the holes provided in core piece 11 as shown in figure 3 and bolt 3b is used to limit vertical movement of the screw 9 since bolt 3b and screw piece 3 clamp core piece 11 as is more clearly shown in figure 2.

By turning the screw piece 3 in a direction indicated by I in figure 4A, the screw 9 will rotate and due to the cooperating threaded surfaces of the screw and the inner surface of the hole 10b of the holding piece 10, said holding piece 10 will travel in a direction II, thereby adjusting the height of the tetrode 2a.

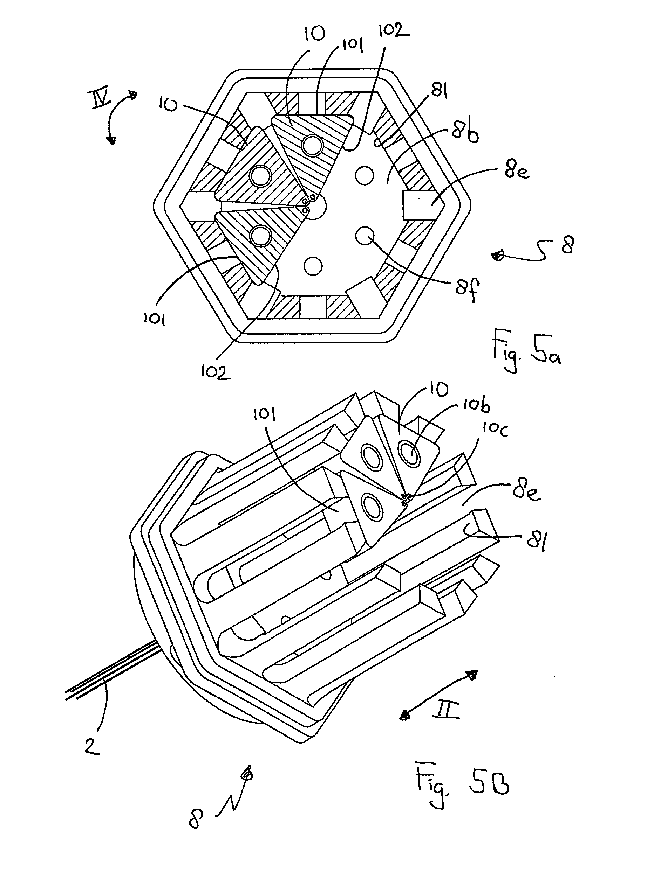

Figure 5A is top view of the body 8 according to the invention showing the assembly of the holding pieces 10 in said body 8. Figure 5B shows the assembly of figure 5B in perspective. Wedge-shaped electrode holding pieces 10 (three of the six are shown in figures 5A and 5B) extend radially and adjacent to each other in circle segments around the axis of the electrode bundle 2. The surfaces 101 of the holding pieces 10 engage inner surface 81 of the body 8, limiting any movement, for instance a wedging movement IV, other than vertical displacement II. The contact surfaces 101 of the holding piece 10 are substantially complementary to the inner surface 81 of the body 8, reducing substantially any margin between said holding pieces 10 and the body 8, resulting in a rectilinear travel path for the electrodes 2a.

Although the radially extending contact surfaces 102 of adjacent electrode holding pieces 10 are spaced apart in figures 5A and 5B, the contact surfaces 102 of another embodiment can be adjacent and engaging, limiting the movement in a direction IV when for instance a body 8 with a smaller inner surface 81 is used.

The aforementioned distance III indicated in figure 4B allows the use of screws 9 with a relative larger diameter with respect to the diameter of the electrodes as can be seen in figure 5A, since more space is available on a radially outward location.

It will be appreciated that there is no need for additional alignment means for the holding pieces 10 as is custom in the prior art. The body and/or the electrode holding pieces themselves provide in the alignment means, allowing substantially rectilinear movement II of the electrodes 2a.

Also clearly visible in figures 5A and 5B are the holes 8f provided in the bottom 8b of the base 8 for receiving the screws 9 and the apertures 8e for reducing the weight of the microdrive 1. In this embodiment, two struts of the body 8 form a contact surface 81 for one holding piece 10.

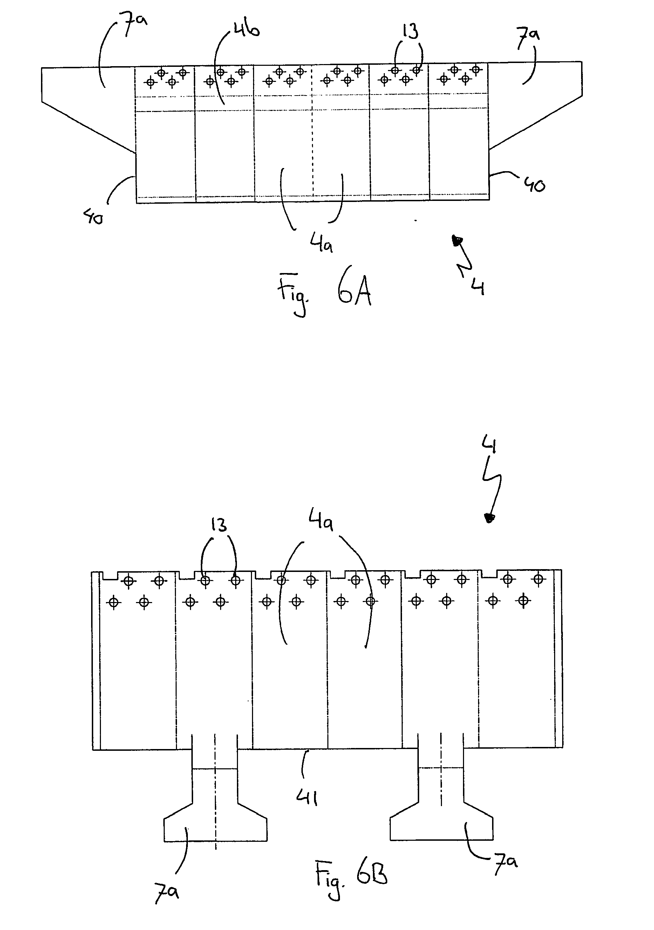

Figures 6a and 6b show layouts of two flexible PCB's 4 according to the invention. The layout shown in figure 6a corresponds to the PCB 4 shown in figure 1. The PCB 4 comprises six planes 4a, corresponding to the hexagonal shape of the body piece 8, which each holds four connector holes 13. Each plane 4a is therefore arranged to connect to four electrodes forming a tetrode 2a. The planes 4b correspond to the height of the core piece 11 when folded over the body piece 8. Since the ends of the electrodes extend above the plane of the core piece 11, said ends of the electrodes can be easily connected to said connector holes 13.

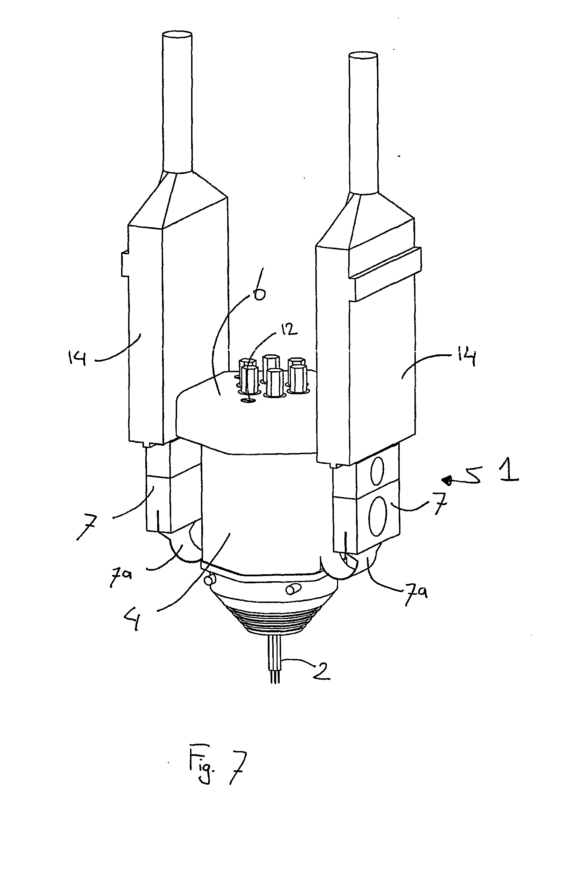

The layout shown in figure 6a comprises two flaps 7a protruding from the edges 40 extending parallel to the axis of the microdrive 1 in the folded state. When wrapped around the body piece 8, flaps 7a form a single connector 7 as is shown in figure 1. The layout of figure 6b however comprises flaps 7a which protrude from a circumferential edge 41 in wrapped state. When wrapped around the body piece 8, the flaps 7a from figure 6b form two separate connectors 7, attached to two conventional headstages, as is shown in figure 7.

The microdrive 1 shown in figure 7 connects to two HS-16 amplifiers 14 (Neuralynx), and can accommodate six tetrodes 2a. The headstage pre-amplifiers 14 are connected to the flexprint 4 via micro-connectors (Omnetics Connectors Corporation, Minneapolis, MN; custom ordered: NPD-18-FF-GS, Nano Dual Row Male, 18 contacts). The connectors 7 are mounted on flaps 7a of the flexprint 4 protruding from the lateral side of the microdrive 1. The upper part of the PCB 4 is fixed in place by cap 6 by screwing the cap 6 on the microdrive 1 using micro-screws 12. The flexprint 4 is lightly glued to the body piece 8 at the bottom, in particular at rims 8d shown in figure 3, with cyanoacrylate.

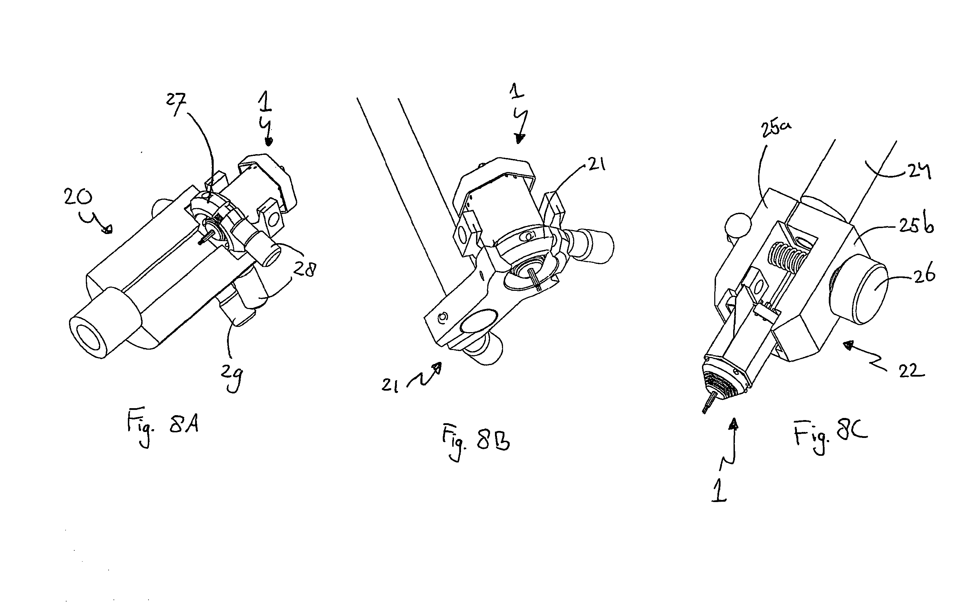

Given the small size of the drive, custom-made tools are necessary for its assembly and surgical implantation. The assembly holder shown in figure 8a can be used during the assembly procedure of the microdrive 1 and was made from Polyacetate (Eriks, Alkmaar, Netherlands) a metal ring 27 that can be fastened onto the drive base piece 5 by means of two set screws 28. The holder 20 allows easy access to the drive top (from where the tetrodes 2a are loaded) and to the bottom part of the drive with the protruding tetrodes 2a. Moreover, the metal ring 27 can be released from the rest of the holder 20 and connected e.g. to a stereotaxic adapter 21 shown in figure 8b, to allow dipping of the tetrodes 2a in the gold-plating solution.

The surgical holder 22 shown in figure 8c can be fit on a standard stereotaxic manipulator 24. It consists of clamps 25a, 25b, operated by a spring-loaded thumb screw 26. After implantation on the animal's head, the holder 22 can be released without applying force on the head.

The present invention is not limited to the embodiments shown, but extends also to other embodiments falling within the scope of the appended claims.

## Claims

1. Microdrive for use in neurophysiological research of animals, particulary for use in mice, comprising:
- at least two elongate electrodes for measuring field potentials from nervous tissue;
- electrode adjusting means for adjusting the depth of each of the electrodes in the nervous tissue; and
- a body for holding the electrode adjusting means,
wherein the electrodes comprise upper parts held in the electrode adjusting means and lower parts forming a bundle arranged for insertion in the animal, **characterized in that** the electrodes extend substantially parallel and linear over their entire length.

2. Microdrive according to claim 1, wherein the electrode adjusting means comprise electrode holding pieces for holding the upper part of an electrode and conveying means for conveying the electrode holding pieces over a predetermined height, wherein the electrode holding pieces are provided with at least one outwardly orientated contact surface for abutting another electrode holding piece and/or the body, thereby fixing the orientation of said electrode holding pieces in the plane perpendicular to the axis of the electrodes.

3. Microdrive according to claim 1 or 2 wherein the electrode holding pieces extend radially and adjacent to each other in circle segments around the electrodes.

4. Microdrive according to claim 3, wherein the electrode holding pieces are substantially wedge-shaped.

5. Microdrive according to any of the preceding claims 2-4, wherein the electrode is held on a radially inward location on the electrode holding piece with respect to and at a distance from the conveying means.

6. Microdrive according to any of the preceding claims 2-5, wherein the conveying means comprise a screw received by the electrode holding piece, wherein relative rotation of the electrode holding piece and the screw results in the adjustment of the height of the electrode holding piece.

7. Microdrive according to claim 6, wherein the screw is rotatable accommodated in the body, wherein rotation of the screw around its longitudinal axis results in the adjustment of the height of the electrode holding piece.

8. Microdrive according to claim 7, wherein the screw is provided with alignment means for substantially limiting movement other than axial rotation.

9. Microdrive according to any of the preceding claims 1 to 8, wherein the body substantially surrounds the electrode adjusting means.

10. Microdrive according to any of the preceding claims 1 to 9, wherein the microdrive comprises a housing and wherein the electrode adjusting means extend outside the housing.

11. Microdrive according to any of the preceding claims 1-10, wherein the microdrive comprises a printed circuit board provided with electrode connectors for connecting with each of the electrodes, wherein the printed circuit board at least partially forms the housing of the microdrive.

12. Microdrive according to claim 11, wherein the electrode connectors are provided near the plane perpendicular to the axis of the electrodes wherein the upper ends of the electrodes extend.

13. Microdrive according to claim 11 or 12, wherein a substantially plate shaped, flexible printed circuit board is folded around the body for forming said housing.

14. Microdrive according to claim 11 or 13, wherein the printed circuit board comprises at least one protrusion which protrudes from the microdrive for forming an external connector for the microdrive.

15. Microdrive according to any of the preceding claims 1 to 14, wherein the microdrive is provided with a base for receiving the bundle of electrodes, wherein the bundle comprises cannulae held in the base for receiving each of the electrodes.

16. Microdrive according to any of the preceding claims 1 to 15, wherein at least the body of the microdrive is manufactured in PEEK.

17. Microdrive according to any of the preceding claims 1 to 16, wherein the electrodes comprise tetrodes.
